# EUROPEAN PATENT APPLICATION

(11) **EP 3 363 364 A1**
(43) Date of publication of application: **22.08.2018**
(21) Application number: 17156873.6
(22) Date of filing: 20.02.2017
(51) Int. Cl.: A61B 6/04, A61B 6/00, G06F 19/00, G06K 9/00

(54) **APPARATUS FOR PROVIDING MAMMOGRAPHY QUALITY ANALYTICS**

(71) Applicant: Koninklijke Philips N.V., 5656 AE Eindhoven (NL)
(72) Inventor: BUELOW, Thomas, 5656 AE Eindhoven (NL); HARDER, Tim Philipp, 5656 AE Eindhoven (NL); NORDHOFF, Tanja, 5656 AE Eindhoven (NL); YOUNG, Stewart, 5656 AE Eindhoven (NL); RICHARD-KOWALSKI, Deborah, 5656 AE Eindhoven (NL)
(74) Representative: de Haan, Poul Erik

(57) **Abstract**

The present invention relates to an apparatus for providing mammography quality analytics. It is described to provide (210) at least one mammogram. A plurality of mammogram acquisition parameters is provided (220), wherein at least one mammogram acquisition parameter is associated with a corresponding mammogram. The at least one mammogram is analysed (230) and a plurality of breast positioning quality parameters is generated, wherein at least one breast positioning quality parameter is associated with a corresponding mammogram. The plurality of mammogram acquisition parameters and the plurality of breast positioning quality parameters is analysed (240) and quality control information is generated (240).

## Description

### FIELD OF THE INVENTION

The present invention relates to an apparatus for providing mammography quality analytics, to a system for providing mammography quality analytics, to a method for providing mammography quality analytics, as well as to a computer program element and a computer readable medium.

### BACKGROUND OF THE INVENTION

The general background of this invention is the mammography. Mammography is the most important imaging method both for screening and for diagnostic workup of breast cancer. High quality of the mammographic image data is a pre-requisite to enable high-quality diagnostic results. Quality assurance and quality control is an important factor for health care providers that not only influences the medical outcome but also has a financial impact (certification, reimbursement). It is currently difficult and labour intensive to obtain an overview of the overall quality of images acquired at an institution. Without this information quality improvement actions cannot be targeted and tailored to the specific needs of the imaging department / institution.

### SUMMARY OF THE INVENTION

It would be advantageous to have improved apparatus providing mammography quality analytics.

The object of the present invention is solved with the subject matter of the independent claims, wherein further embodiments are incorporated in the dependent claims. It should be noted that the following described aspects and examples of the invention apply also for the apparatus providing mammography quality analytics, the system providing mammography quality analytics, the method providing mammography quality analytics, and for the computer program element and the computer readable medium.

According to a first aspect, there is provided an apparatus for providing mammography quality analytics, comprising:
- an input unit; and
- a processing unit.

The input unit is configured to provide the processing unit with at least one mammogram. The input unit is also configured to provide the processing unit with a plurality of mammogram acquisition parameters, wherein at least one mammogram acquisition parameter is associated with a corresponding mammogram. The processing unit is configured to implement a positioning assessment module to analyse the at least one mammogram and generate a plurality of breast positioning quality parameters. At least one breast positioning quality parameter is associated with a corresponding mammogram. The processing unit is also configured to implement a quality control assessment module to analyse the plurality of mammogram acquisition parameters and the plurality of breast positioning quality parameters and generate quality control information.

In other words, the image quality of mammograms is determined in terms of the quality of the positioning of the breast during acquisition of the mammogram, and this is correlated with associated acquisition parameters such as the operator who took the image, how long the operator had been working, time of day, whether the right or left breast was imaged, and characteristics of the patient such as age, body mass index etc.

Thus, a large number of image acquisition parameters can be analysed along with determined or generated quality parameters relating to breast positioning, to provide a statistical insight into where positional quality is non-optimum and how that can be remedied.

In this manner, it can be automatically determined if remedial action is required, and where the deficiency in breast positioning is to be found. Thus, improvement actions can be automatically generated, which could be operator specific or apply to specific characteristics of patients or that all operators should have a break after a certain amount of time working for example.

Thus, an operator when setting up a patient for mammography is automatically provided with bespoke information that relates to them and/or the type of patient under examination, enabling them to more correctly position the breast for the mammogram.

In an example, the processing unit is configured to implement a root cause analysis module as part of the quality control assessment module. The root cause analysis module is configured to determine at least one repetitive pattern in the plurality of breast positioning quality parameters as part of the generation of the quality control information.

In other words, quality control measurements and statistical evaluation of a set of mammographic examinations enables the determination of repetitive patterns in image quality issues, and these issues can be linked to their root causes. Thus, remedial action can be generated in the form of quality control information enabling for improvement in terms of the actions to be performed by the operator.

In an example, the processing unit is configured to implement an action module to analyse the quality control information and generate breast positioning information.

In this manner, an operator can address issues associated with a particular type of patient, for example taking into account body mass index, and can take account of their own deficiencies in terms of the positioning of breasts during a mammogram. Thus, an overall improvement in the acquisition of mammograms is facilitated.

In an example, the plurality of acquisition parameters comprises X-ray equipment operator information.

Thus, specific issues can be identified for specific operators, and bespoke rectifying actions can be provided for operators.

In an example, the plurality of acquisition parameters comprises one or more of: time of day; day of week; compression force on the breast; patient characteristics; and whether a mammogram relates to a right or left breast.

In this way, imaging issues can be identified that could relate to global issues such as the time of day that could affect all operators, or only some operators, and whether for example some operators position one breast better than another.

In this manner, generated breast positioning quality parameters can be used to predict the outcome for a given set of boundary conditions (BMI, patient age, time of day. Operator ID etc) and individualized suggestions for attention points can be derived.

In an example, the at least one mammogram comprises at least one medio-lateral oblique (MLO) image, and wherein the plurality of breast positioning quality parameters comprises one or more of: whether the pectoral muscle is shown to nipple level; the angle of the pectoral muscle; whether the angle of the pectoral muscle is greater than 20 degrees; whether the nipple is shown in profile; whether the infra-mammary angle is clearly demonstrated; whether all the breast tissue is clearly shown; whether the inferior pectoralis extent is greater than zero; and when the at least one mammogram comprises mammograms of the right and left breast of the same person whether the right and left mammograms are symmetric.

In an example, the at least one mammogram comprises at least one cranio-caudal (CC) image, and wherein the plurality of breast positioning quality parameters comprises one or more of: whether the nipple is shown in profile; the extent to which the lateral aspect of the breast is shown; whether the pectoral muscle shadow is shown on the posterior edge of the breast; whether the medial border of the breast is shown; and when the at least one mammogram comprises mammograms of the right and left breast of the same person whether the right and left mammograms are symmetric.

In an example, the at least one mammogram comprises at least one medio-lateral oblique (MLO) image and at least one cranio-caudal (CC) image of the same breast, and wherein the plurality of breast positioning quality parameters comprises a difference in a distance from the nipple to the posterior edge in a CC image to a distance from the nipple to the pectoral muscle in the MLO image.

In an example, the plurality of breast positioning quality parameters comprises whether the difference in distance is less than 10mm.

According to a second aspect, there is provided a system for providing mammography quality analytics, comprising:
- at least one information providing unit;
- an apparatus for providing mammography quality analytics according to the first aspect; and
- an output unit

The at least one mammogram is provided from the at least one information providing unit to the input unit. The plurality of mammogram acquisition parameters is provided from the at least one information providing unit to the input unit. The output unit is configured to output the quality control information.

According to a third aspect, there is provided a method for providing mammography quality analytics, comprising:
- providing at least one mammogram;
- providing a plurality of mammogram acquisition parameters, wherein at least one mammogram acquisition parameter is associated with a corresponding mammogram;
- analysing the at least one mammogram and generating a plurality of breast positioning quality parameters, wherein at least one breast positioning quality parameter is associated with a corresponding mammogram;
- analysing the plurality of mammogram acquisition parameters and the plurality of breast positioning quality parameters and generating quality control information.

In an example, step d) comprises determining at least one repetitive pattern in the plurality of breast positioning quality parameters as part of generating the quality control information.

In an example, the method comprises step e), the step comprising analysing the quality control information and generating breast positioning information.

According to another aspect, there is provided a computer program element controlling apparatus as previously described which, if the computer program element is executed by a processing unit, is adapted to perform the method steps as previously described.

According to another aspect, there is provided a computer readable medium having stored computer element as previously described.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for providing mammography quality analytics;
Fig. 2 shows a schematic set up of an example of a system for providing mammography quality analytics;
Fig. 3 shows a method for providing mammography quality analytics;
Fig. 4 shows two mammographic views of the same breast, one a medio-lateral oblique (MLO) view and the other a cranio-caudal (CC) view;
Fig. 5 shows an example of a mammography quality dashboard provided by an example of an apparatus for providing mammography quality analytics;
Fig. 6 shows an example of a mammography quality dashboard provided by an example of an apparatus for providing mammography quality analytics;
Fig. 7 shows different levels of quality analytics; and
Fig 8 shows a feed forward neural network with a single hidden layer.

### DETAILED DESCRIPTION OF EMBODIMENTS

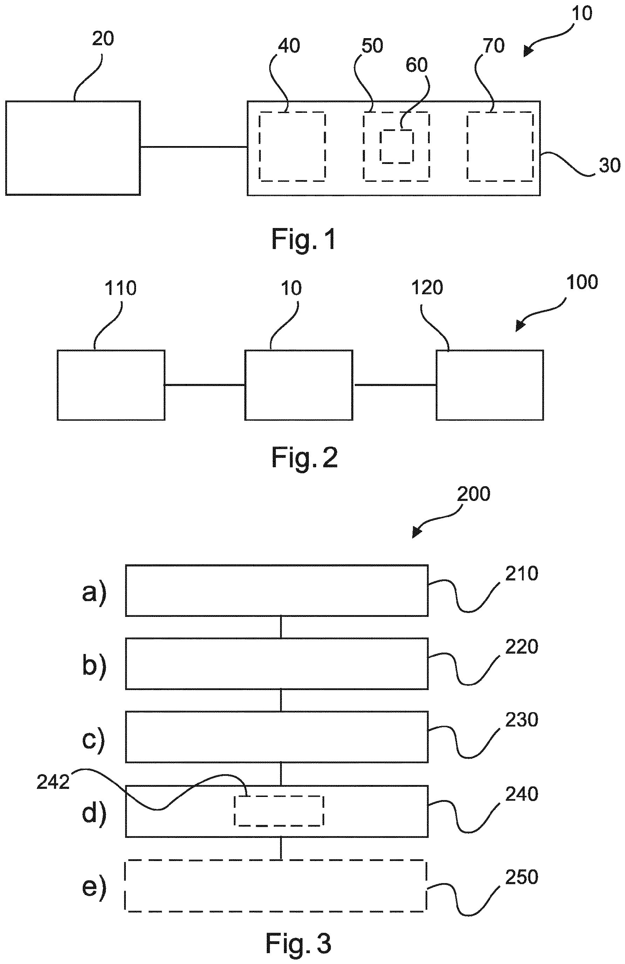

Fig. 1 shows an example of an apparatus 10 for providing mammography quality analytics. The apparatus 10 comprises an input unit 20 and a processing unit 30. The input unit 20 is configured to provide the processing unit 30 with at least one mammogram. This is done via wired or wireless communication. The input unit 20 is also configured to provide the processing unit 30 with a plurality of mammogram acquisition parameters. This is done via wired or wireless communication. The at least one mammogram acquisition parameter is associated with a corresponding mammogram. The processing unit 30 is configured to implement a positioning assessment module 40 to analyse the at least one mammogram and generate a plurality of breast positioning quality parameters. At least one breast positioning quality parameter is associated with a corresponding mammogram. The processing unit 30 is also configured to implement a quality control assessment module 50 to analyse the plurality of mammogram acquisition parameters and the plurality of breast positioning quality parameters and generate quality control information.

In an example, the plurality of mammogram acquisition parameters for a mammogram is intrinsically associated with the image data, for example is to be found in the Digital Imaging and Communications in Medicine (DICOM) header.

In an example, the positioning assessment module comprises algorithms for the automatic evaluation of the quality of mammograms, for example as described in the following paper: Thomas Bülow, Kirsten Meetz, Dominik Kutra, Thomas Netsch, Rafael Wiemker, Martin Bergtholdt, Jörg Sabczynski, Nataly Wieberneit, Manuela Freund, and Ingrid Schulze-Wenck. "Automatic assessment of the quality of patient positioning in mammography." In SPIE Medical Imaging, pp. 867024-867024. International Society for Optics and Photonics, 2013.

According to an example, the processing unit 30 is configured to implement a root cause analysis module 60 as part of the quality control assessment module 50. The root cause analysis module 60 is configured to determine at least one repetitive pattern in the plurality of breast positioning quality parameters as part of the generation of the quality control information.

In an example, the root cause analysis module is configured determine a reason (root cause) for the pattern of deficiencies to occur, on the basis of the plurality of mammogram acquisition parameters and the plurality of breast positioning quality parameters. In other words, a correlation is provided to a reason or reasons (root cause(s)) for the pattern of deficiencies to occur.

In an example, the root cause analysis module is configured to determine if a generated breast positioning quality parameter is deficient, based on a comparison of a generated breast positioning quality parameter with ground truth information. In an example, the root cause analysis module is configured to identify the reason for this quality parameter being deficient, e.g., something the operator did not do correctly.

In an example, such ground truth information is provided through medical personal reviewing a number of mammograms and providing feedback relating to the positioning of the breast during the mammogram, or other mammogram acquisition parameters such as the compression pressure applied was too low or too high.

According to an example, the processing unit 30 is configured to implement an action module 70 to analyse the quality control information and generate breast positioning information.
According to an example, the plurality of acquisition parameters comprises X-ray equipment operator information.

In an example, the operator information includes the identity of the operator. In an example, the operator information includes how long the operator has been working in a shift. In an example, the operator information includes how many mammograms the operator has taken.

According to an example, the plurality of acquisition parameters comprises one or more of: time of day; day of week; compression force on the breast; patient characteristics; and whether a mammogram relates to a right or left breast.

In an example, patient characteristics includes body mass index (BMI). In an example, patient characteristics includes body part thickness.

According to an example, the at least one mammogram comprises at least one medio-lateral oblique (MLO) image, and the plurality of breast positioning quality parameters comprises one or more of: whether the pectoral muscle is shown to nipple level; the angle of the pectoral muscle; whether the angle of the pectoral muscle is greater than 20 degrees; whether the nipple is shown in profile; whether the infra-mammary angle is clearly demonstrated; whether all the breast tissue is clearly shown; whether the inferior pectoralis extent is greater than zero; and when the at least one mammogram comprises mammograms of the right and left breast of the same person whether the right and left mammograms are symmetric.

According to an example, the at least one mammogram comprises at least one cranio-caudal (CC) image, and the plurality of breast positioning quality parameters comprises one or more of: whether the nipple is shown in profile; the extent to which the lateral aspect of the breast is shown; whether the pectoral muscle shadow is shown on the posterior edge of the breast; whether the medial border of the breast is shown; and when the at least one mammogram comprises mammograms of the right and left breast of the same person whether the right and left mammograms are symmetric.

According to an example, the at least one mammogram comprises at least one medio-lateral oblique (MLO) image and at least one cranio-caudal (CC) image of the same breast, and the plurality of breast positioning quality parameters comprises a difference in a distance from the nipple to the posterior edge in a CC image to a distance from the nipple to the pectoral muscle in the MLO image.

According to an example, the plurality of breast positioning quality parameters comprises whether the difference in distance is less than 10mm.

Fig. 2 shows an example of a system 100 for providing mammography quality analytics. The system 100 comprises at least one information providing unit 110, an apparatus 10 for providing mammography quality analytics as described with respect to Fig. 1, and an output unit 120. The at least one mammogram is provided from the at least one information providing unit to the input unit. This is done via wired or wireless communication. The plurality of mammogram acquisition parameters is provided from the at least one information providing unit to the input unit. This is done via wired or wireless communication. The output unit is configured to output the quality control information.

In an example, the information providing unit is an information storage device, such as a database

Fig. 3 shows a method 200 for providing mammography quality analytics in its basic steps. The method 200 comprises:
- in a providing step 210, also referred to as step a), providing at least one mammogram;
- in a providing step 220, also referred to as step b), providing a plurality of mammogram acquisition parameters, wherein at least one mammogram acquisition parameter is associated with a corresponding mammogram;
- in an analyzing and generating step 230, also referred to as step c), analysing the at least one mammogram and generating a plurality of breast positioning quality parameters, wherein at least one breast positioning quality parameter is associated with a corresponding mammogram;
- in an analyzing and generating step 240, also referred to as step d), analysing the plurality of mammogram acquisition parameters and the plurality of breast positioning quality parameters and generating quality control information.

In an example, step a) comprises providing the at least one mammogram from an input unit to a processing unit.

In an example, step b) comprises providing the plurality of mammogram acquisition parameters from the input unit to the processing unit.

In an example, step c) comprises the processing unit implementing a positioning assessment module.

In an example, step d) comprises the processing unit implementing a quality control assessment module.

According to an example, step d) comprises determining 242 at least one repetitive pattern in the plurality of breast positioning quality parameters as part of generating the quality control information.

According to an example, the method comprises step e), the step comprising analysing 150 the quality control information and generating breast positioning information. In an example, step e) comprises the processing unit implementing an action module.
In an example, the plurality of acquisition parameters comprises X-ray equipment operator information.

In an example, the plurality of acquisition parameters comprises one or more of: time of day; day of week; compression force on the breast; patient characteristics; and whether a mammogram relates to a right or left breast.

In an example, the at least one mammogram comprises at least one medio-lateral oblique (MLO) image, and the plurality of breast positioning quality parameters comprises one or more of: whether the pectoral muscle is shown to nipple level; the angle of the pectoral muscle; whether the angle of the pectoral muscle is greater than 20 degrees; whether the nipple is shown in profile; whether the infra-mammary angle is clearly demonstrated; whether all the breast tissue is clearly shown; whether the inferior pectoralis extent is greater than zero; and when the at least one mammogram comprises mammograms of the right and left breast of the same person whether the right and left mammograms are symmetric.

In an example, the at least one mammogram comprises at least one cranio-caudal (CC) image, and the plurality of breast positioning quality parameters comprises one or more of: whether the nipple is shown in profile; the extent to which the lateral aspect of the breast is shown; whether the pectoral muscle shadow is shown on the posterior edge of the breast; whether the medial border of the breast is shown; and when the at least one mammogram comprises mammograms of the right and left breast of the same person whether the right and left mammograms are symmetric.

In an example, the at least one mammogram comprises at least one medio-lateral oblique (MLO) image and at least one cranio-caudal (CC) image of the same breast, and wherein the plurality of breast positioning quality parameters comprises a difference in a distance from the nipple to the posterior edge in a CC image to a distance from the nipple to the pectoral muscle in the MLO image.

In an example, the plurality of breast positioning quality parameters comprises whether the difference in distance is less than 10mm.

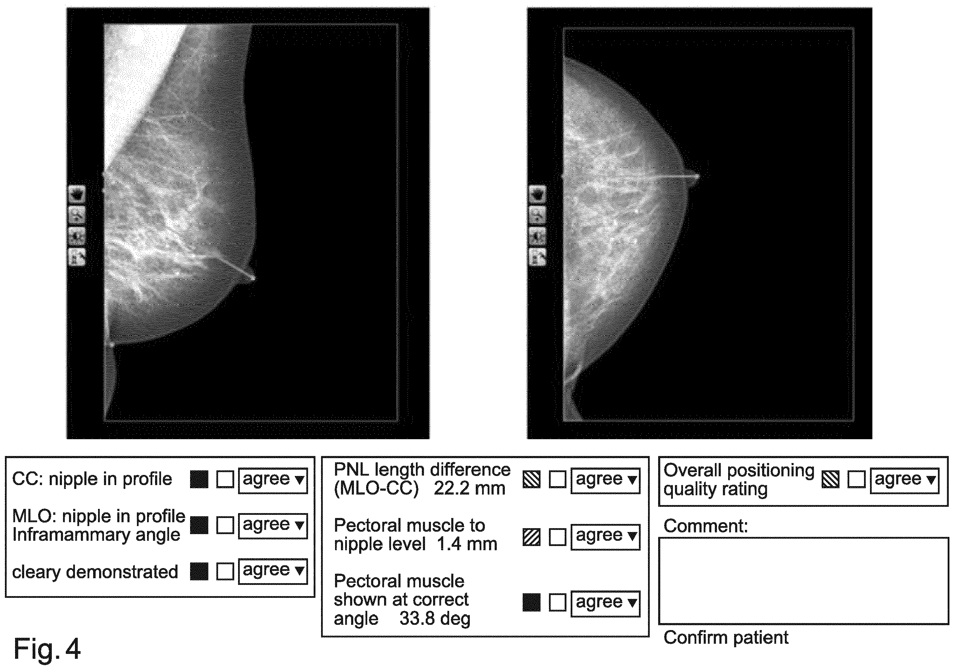

The apparatus, system and method for providing mammography quality analytics are now described in more detail in conjunction with Figs. 4-8 and Tables 1, 2 and 3, which are appended below.

Quality assurance and control in relation to mammography is a time consuming task, which is currently performed visually by human observers reading individual imaging exams. In this current scheme, it is not practically feasible to obtain an overview of the overall quality of images acquired at an institution. Without this information quality improvement actions cannot be targeted and tailored to the specific needs of the imaging department / institution. The current manual analysis is particularly unsuited for continuous monitoring and improvement of image quality due to the time consuming assessment process.

The presently described apparatus, system and method for providing mammography quality analytics address these issues. In detail, with respect to the system, where the system has a module for automatic analysis of the positioning quality of mammograms based on generally accepted clinical quality criteria, and this is combined with quality control parameters available from the DICOM header and its application on a large scale (PACS-level). The resulting quality information can be reported to the user in a cumulative fashion, e.g., aggregated over a certain time interval, including interactive data visualization that allows the user to inspect correlation of quality with external factors such as operator, time of day, left vs. right breast, etc. A root-cause-analysis module automatically generates improvement action proposals to be performed by the operator in an upcoming examination, given information on time of day, performing operator or even patient characteristics for example.

Thus, elements of the system are as detailed below:
- A mechanism for automatic analysis of the quality of mammograms with respect to positioning, as well as additional information available from the DICOM header, based on generally accepted clinical quality criteria.
- A mechanism for automatic analysis according to the above criteria combined with technical quality control measurements and statistic evaluation of a set of mammographic examinations allowing for analysis of the data with respect to repetitive patterns in image quality issues (This can be termed - Descriptive Analytics).
- A mechanism for reporting of overall image quality, e.g., aggregated over a certain time interval, including interactive data visualization that allows the user to inspect correlation of quality with external factors such as operator, time of day, left vs. right breast, etc. This information can be used to derive the expected outcome given a set of known boundary conditions / external factors. (This can be termed - Predictive Analytics).
- A root-cause-analysis mechanism linking observed issues to their root-causes (This can be termed - Diagnostic Analytics).
- An automatic generation mechanism, for generating feedback, instructions and/or suggestions for improvement in terms of actions to be performed by the operator. (This can be termed - Prescriptive Analytics).

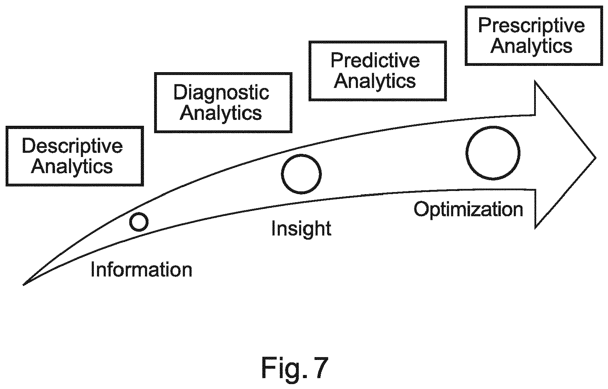

The different levels of quality analytics are shown in Fig. 7.

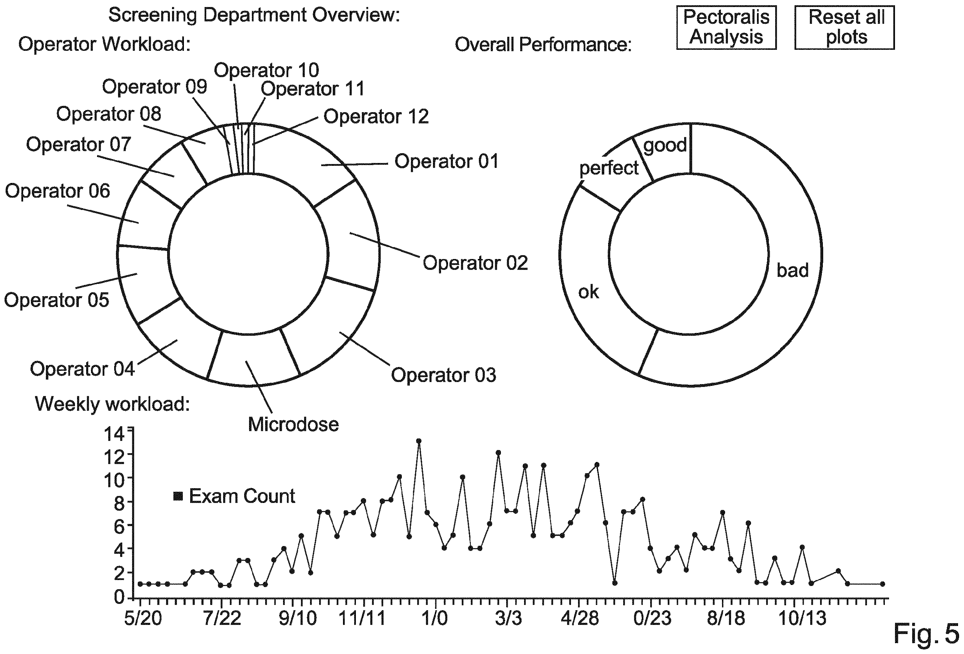

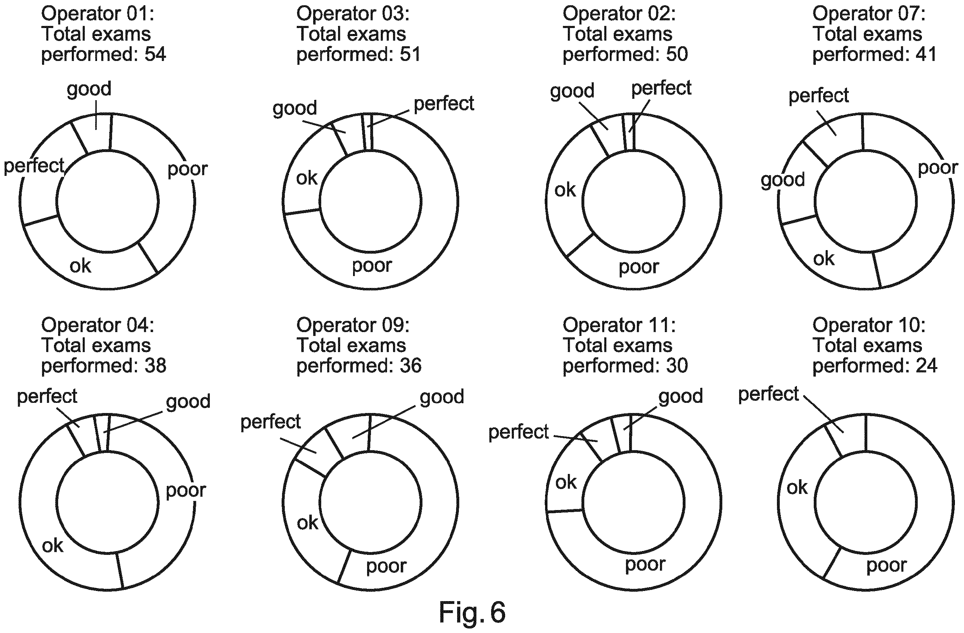

The following is a detailed workflow, for providing mammography quality analytics:
- As a first step algorithms for the automatic evaluation of the quality of mammograms are applied to those mammograms. Examples of such algorithms are discussed in Bülow et al, referred to above. The mammograms provided are in the MLO and CC views, as shown in Fig. 4. The evaluated quality criteria can include, but are not limited to the features, shown in Tables 1A and 1B below.
- The various quality criteria are automatically evaluated on a set of mammograms, e.g., data from past quarter/past year of a screening centre allowing for analysis of the data with respect to repetitive patterns in image quality issues.
- A Mammography Quality Database (MQD) is set up to hold data including:
   - Results for the different positioning quality criteria
   - Technical quality measures derived from regular quality control measurements and phantom measurements (ACR Phantom analysis), CNR
   - Performance measures such as Repeat / Reject analysis
   - Secondary quality measures and additional information available from the images' DICOM headers, such as:
      - Compression force and body part thickness
      - Acquisition time and date
      - Operator name/ID
- A dictionary linking deficient image quality criteria to root causes and actions for improvement is established (See Table 2)
- An analytics engine is applied to the MQD to generate representative reports such as the ones shown in Figs. 4 and 5. Fig. 5 shows an example of a Mammography quality dashboard: For a given period of time the distribution of scans over the operators is displayed (upper left), and the distribution of scan quality (upper right) as well as the number of scans per time interval is presented (graph on the bottom). Fig. 6 shows another example of a Mammography quality dashboard: here the quality distributions are presented per operator.
- Quality features can be analysed and presented on an individual operator level, or on an aggregated level.
- Overall quality and individual quality features can be plotted separately by operator, comparing results for left breast vs. right breasts, grouped by patients' BMIs, by day of the week, by compression force etc.
- The descriptive quality analysis of the previous step is used to predict the outcome for a given set of boundary conditions (BMI, patient age, time of day, operator ID) and derives individualized suggestions for attention points: for example "For the next patient, please pay special attention to pull down the abdomen in order to clearly image the infra-mammary fold."
- Combining the results of the quality analysis with the link to root-causes, suggestions for improvement and training actions can automatically be derived. Examples for possible improvement suggestions can include
   - "Operator x should pay attention to lift the breast when positioning for an MLO view of the right breast"
   - "As quality tends to degrade over time, operators should not work more than 60 minutes without break"
- After having established a base-line assessment along with proposed improvement actions, the image quality can be monitored on an on-going basis using the workflow described above.

The different levels of Quality Analytics are visualized in Fig 7. The four levels of quality analytics referred to above are pictorially represented, and this figure gives a visual summary of the apparatus, system and method for providing mammography quality analytics. As represented in Fig. 7, during descriptive analytics mammography quality is measured, aggregated and reported. Linking observed quality deficiencies to the respective root-causes is part of diagnostic analytics. Predictive analytics uses this information to predict the outcome under given boundary conditions. Deriving specific suggestions for actions and attention points for the user is part of the final stage of prescriptive analytics.

### Linking Image Features to Root Causes

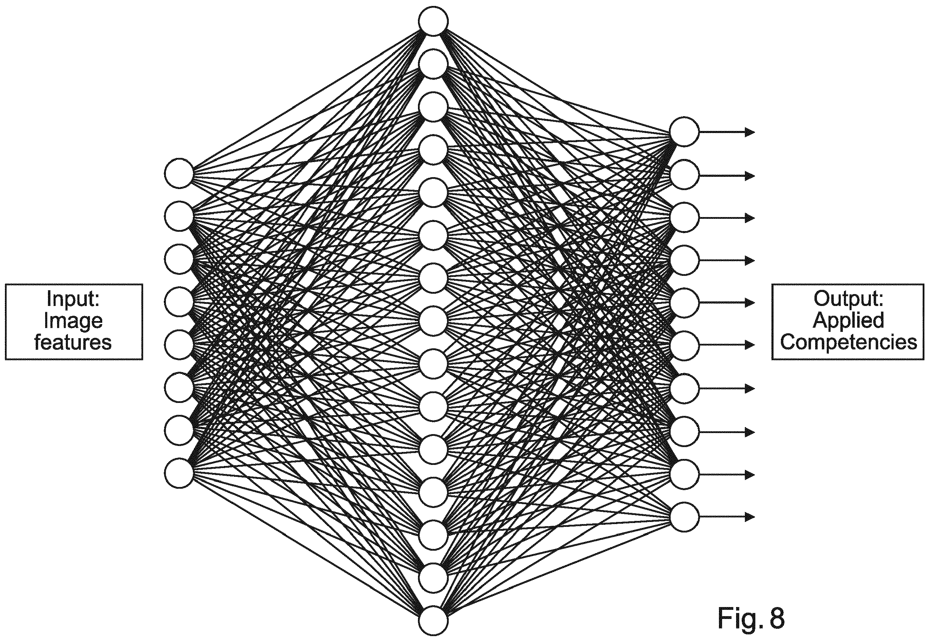

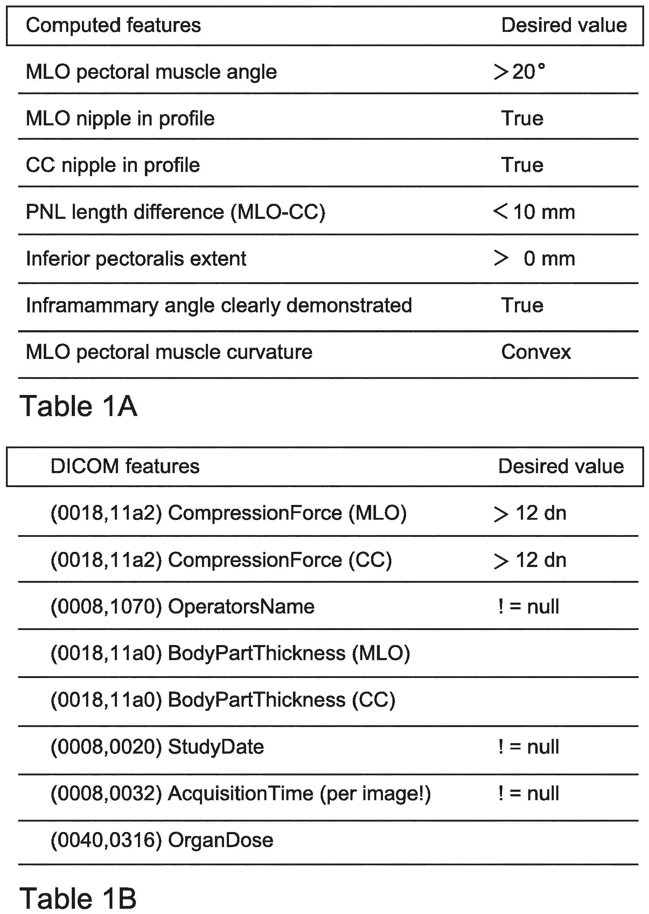

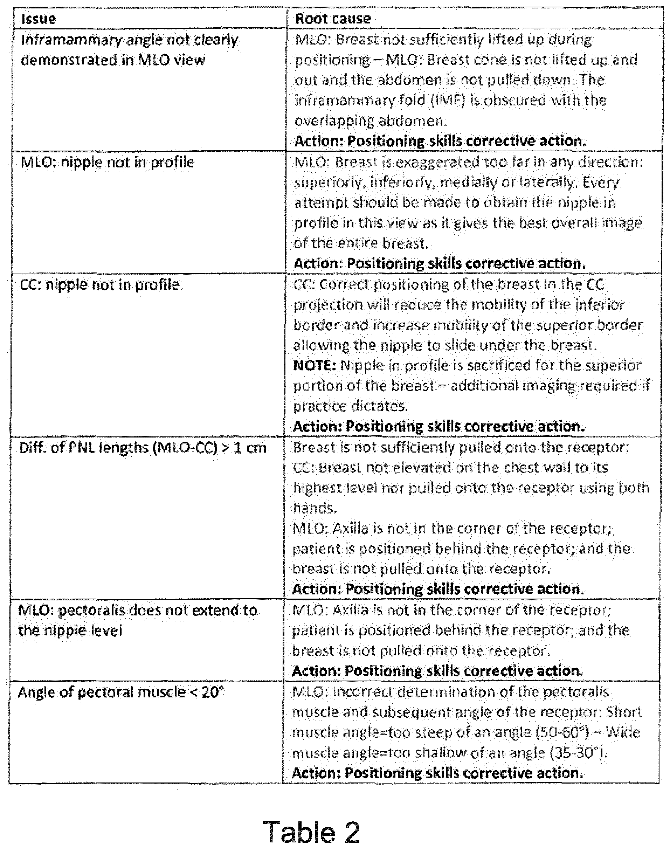

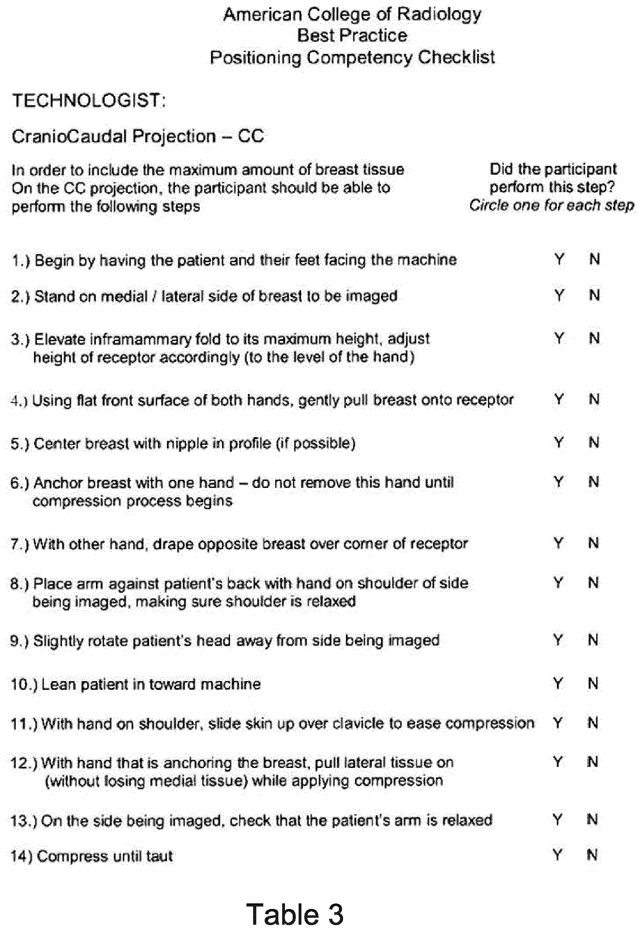

Further detail is now provided relating to the root cause analysis module. Table 3 shows a check list for assessing a technologist's competencies with respect to patient positioning in mammography. Failing on one or more of these competencies can be considered the root cause for a sub-optimal mammogram. The collection of data according to this check-list, in addition to imaging information, provides the data needed to train a pattern recognition system designed for the prediction of missing/incorrectly performed steps in the positioning procedure from the resulting mammogram. Fig. 8 shows a symbolic representation of a pattern recognition technology used within the root cause analysis module that performs this, which in this example is shown as a feed-forward neural network with a single hidden layer. Input to the pattern recognition system would are the image features according to Table 1, and the output is a vector representing the competency check-list. Regardless of the visual representation as a neural network, other pattern recognition algorithms such as support vector machines (SVM) can be trained for this task. Training such a system requires a significant amount of data in order to be statistically reliable (>1000 mammograms + competency information). In return the gained insight into the root-causes of certain image deficiencies from such an extensive amount of data is extremely difficult for even a skilled practitioner to attempt to deduce.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

**1.** An apparatus (10) for providing mammography quality analytics, comprising:
- an input unit (20); and
- a processing unit (30);
wherein, the input unit is configured to provide the processing unit with at least one mammogram;
wherein, the input unit is configured to provide the processing unit with a plurality of mammogram acquisition parameters, wherein at least one mammogram acquisition parameter is associated with a corresponding mammogram;
wherein, the processing unit is configured to implement a positioning assessment module (40) to analyse the at least one mammogram and generate a plurality of breast positioning quality parameters, wherein at least one breast positioning quality parameter is associated with a corresponding mammogram;
wherein, the processing unit is configured to implement a quality control assessment module (50) to analyse the plurality of mammogram acquisition parameters and the plurality of breast positioning quality parameters and generate quality control information.

**2.** Apparatus according to claim 1, wherein the processing unit (30) is configured to implement a root cause analysis module (60) as part of the quality control assessment module (50), and wherein the root cause analysis module is configured to determine at least one repetitive pattern in the plurality of breast positioning quality parameters as part of the generation of the quality control information.

**3.** Apparatus according to any of claims 1-2, wherein the processing unit (30) is configured to implement an action module (70) to analyse the quality control information and generate breast positioning information.

**4.** Apparatus according to any of claims 1-3, wherein the plurality of acquisition parameters comprises X-ray equipment operator information.

**5.** Apparatus according to any of claims 1-4, wherein the plurality of acquisition parameters comprises one or more of: time of day; day of week; compression force on the breast; patient characteristics; and whether a mammogram relates to a right or left breast.

**6.** Apparatus according to any of claims 1-5, wherein the at least one mammogram comprises at least one medio-lateral oblique (MLO) image, and wherein the plurality of breast positioning quality parameters comprises one or more of: whether the pectoral muscle is shown to nipple level; the angle of the pectoral muscle; whether the angle of the pectoral muscle is greater than 20 degrees; whether the nipple is shown in profile; whether the infra-mammary angle is clearly demonstrated; whether all the breast tissue is clearly shown; whether the inferior pectoralis extent is greater than zero; and when the at least one mammogram comprises mammograms of the right and left breast of the same person whether the right and left mammograms are symmetric.

**7.** Apparatus according to any of claims 1-6, wherein the at least one mammogram comprises at least one cranio-caudal (CC) image, and wherein the plurality of breast positioning quality parameters comprises one or more of: whether the nipple is shown in profile; the extent to which the lateral aspect of the breast is shown; whether the pectoral muscle shadow is shown on the posterior edge of the breast; whether the medial border of the breast is shown; and when the at least one mammogram comprises mammograms of the right and left breast of the same person whether the right and left mammograms are symmetric.

**8.** Apparatus according to any of claims 1-7, wherein the at least one mammogram comprises at least one medio-lateral oblique (MLO) image and at least one cranio-caudal (CC) image of the same breast, and wherein the plurality of breast positioning quality parameters comprises a difference in a distance from the nipple to the posterior edge in a CC image to a distance from the nipple to the pectoral muscle in the MLO image.

**9.** Apparatus according to claim 8, wherein the plurality of breast positioning quality parameters comprises whether the difference in distance is less than 10mm.

**10.** A system (100) for providing mammography quality analytics, comprising:
- at least one information providing unit (110);
- an apparatus (10) for providing mammography quality analytics according to any of claims 1-9; and
- an output unit (120);
wherein, the at least one mammogram is provided from the at least one information providing unit to the input unit;
wherein, the plurality of mammogram acquisition parameters is provided from the at least one information providing unit to the input unit;
wherein, the output unit is configured to output the quality control information.

**10.** A method (200) for providing mammography quality analytics, comprising:
- providing (210) at least one mammogram;
- providing (220) a plurality of mammogram acquisition parameters, wherein at least one mammogram acquisition parameter is associated with a corresponding mammogram;
- analysing (230) the at least one mammogram and generating a plurality of breast positioning quality parameters, wherein at least one breast positioning quality parameter is associated with a corresponding mammogram;
- analysing (240) the plurality of mammogram acquisition parameters and the plurality of breast positioning quality parameters and generating quality control information.

**11.** Method according to claim 11, wherein step d) comprises determining (242) at least one repetitive pattern in the plurality of breast positioning quality parameters as part of generating the quality control information.

**12.** Method according to any of claims 11-12, comprising step e), analysing (150) the quality control information and generating breast positioning information.

**13.** A computer program for controlling an apparatus according to one of claims 1 to 9 and/or system of claim 10, which when executed by a processor is configured to carry out the method of any one of claims 11 to 13.

**14.** A computer readable medium having stored the program element of claim 14.
